# EUROPEAN PATENT APPLICATION

(11) **EP 4 282 315 A1**
(43) Date of publication of application: **29.11.2023**
(21) Application number: 22175215.7
(22) Date of filing: 24.05.2022
(51) Int. Cl.: A61B 1/00, A61B 1/05, G02B 23/24, A61B 1/06

(54) **ENDOSCOPE COMPRISING A DISTAL TIP UNIT WITH A CAMERA MODULE**

(71) Applicant: Ambu A/S, 2750 Ballerup (DK)
(72) Inventor: SØRENSEN, Morten, 2750 Ballerup (DK)
(74) Representative: Winter, Brandl - Partnerschaft mbB

(57) **Abstract**

The present disclosure relates to an endoscope (2) comprising: a proximal endoscope handle (4) or interface; and an insertion cord (6) configured to be inserted into a patient's body cavity and comprising a modular distal tip unit (12); the modular distal tip unit (12) comprising: a main body (40) connected to a remainder of the insertion cord (6); and a camera module (42) configured to be accommodated and inserted in a receptacle (46) formed in the main body (40), the camera module (42) comprising a non-cylindrical housing (48) defining an internal space (96) in which at least a light emitting device (14) and an imaging device (16) are accommodated.

## Description

The present disclosure relates to an endoscope, in particular single-use endoscope, comprising a proximal endoscope handle or interface and an insertion cord configured to be inserted into a patient's body cavity and comprising a distal tip unit, in which a light emitting device and an imaging device are accommodated.

### Related art

Endoscopes and similar specialized instruments such as bronchoscopes, arthroscopes, colonoscopes, laparoscopes, gastroscopes, duodenoscopes and ureteroscopes are well known from the state of the art and are used for visual examination and diagnosis of hollow organs and body cavities, as well as to assist in surgery, e.g. for a targeted tissue sampling. Both reusable and disposable (i.e. single-use) endoscopes are known from the related art. Known endoscopes usually comprise an endoscope handle via which an operator/ user can hold and control the endoscope. An insertion cord comprising an insertion tube, an actively bendable bending section and a distal tip unit is usually connected to the endoscope handle. The insertion cord is configured to be inserted into the hollow organs and body cavities of a patient. The distal tip unit usually contains a light emitting device like a light-emitting diode or a fiber optic light guide connected to a proximal source of light and an imaging device comprising e.g. an image/camera sensor and a lens system, in particular a lens barrel, so that the patient's body cavity can be illuminated and viewed by the operator/ user, e.g. via a monitor connected to the endoscope.

Reusable endoscopes are basically very heavy, complex in design and expensive, and need to be reprocessed, in particular sterilized, after each use. Since the distal tip unit usually comprises the mentioned sensitive electrical and optical elements, sterilization possibilities for reusable endoscopes are basically limited. E.g., sterilization in an autoclave usually requires high temperatures and high pressures, which could destroy or wear the camera sensor. Using more gentle sterilization procedures like chemical procedures may however result in incomplete sterilization and thus in an increased danger of cross-contamination.

DE 198 06 984 A1 tries to deal with this problem and provides a detachable distal tip unit which can be easily detached due to sterilization purposes. The detachable distal tip unit comprises a cylindrical housing and a cylindrical cover surrounding the cylindrical housing and being rotatable and axially displaceable with respect to the cylindrical housing. The cylindrical housing is connected to a mounting part of the remainder of the insertion cord via a protrusion engaging in a recess of the mounting part and via electrical contacts. The cylindrical cover presses a flange of the cylindrical housing against the mounting part and is connected to the mounting part via a screw connection. After a use of the endoscope disclosed in DE 198 06 984 A1 the detachable distal tip unit is sterilized in a chemical sterilization procedure and the remainder of the endoscope is sterilized in an autoclave. The above-mentioned disadvantages of reusable endoscopes are thus at least partially mitigated by the endoscope disclosed in DE 198 06 984 A1. However, the endoscope disclosed in DE 198 06 984 A1 is still heavy, complex in design and expensive, and there remains still a certain risk of incomplete sterilization in particular of the detachable distal tip unit and thus the danger of cross-contamination.

According to the present disclosure, it is therefore preferred to provide a single-use endoscope, i.e. an endoscope, which is preferably low-cost, lightweight and intended to be disposed after use. WO 2021/219801 A1 e.g. already discloses a distal tip unit of a single-use endoscope. The distal tip unit disclosed in WO 2021/219801 A1 comprises a first housing part attached to a remainder of the insertion cord and a second housing part connected to the first housing part. In an inner cavity of a housing formed by the first housing part and the second housing part a printed circuit board is arranged, and a light emitting device and an imaging device are electrically connected to the printed circuit board. When assembling the endoscope disclosed in WO 2021/219801 A1, the printed circuit board, the light emitting device and the imaging device are inserted into the housing and the first and second housing parts are fluid-tightly connected to each other. After that, the inner cavity of the housing is filled with an opaque potting, so that the electrical and optical elements inside the housing are potted by means of a potting compound like a potting glue. This makes it possible that the mounting respectively the position of the electrical and optical elements inside the housing is frozen and that the electrical and optical elements are liquid-tightly accommodated or framed by the potting. Further, light is prevented from reaching the imaging device.

The endoscope disclosed in WO 2021/219801 A1 however has the disadvantage that testing of the distal tip unit, in particular of the light emitting device and the imaging device, for liquid-tightness, quality and functionality can only be performed when the single-use endoscope is already entirely assembled. Disadvantageously, in case the single-use endoscope disclosed in WO 2021/219801 A1 does not pass a certain test like a test for liquid-tightness after its assembly due to a certain defect, the entire finished and assembled endoscope has to be discarded. Moreover, the assembly of the endoscope, in particular of the distal tip unit, disclosed in WO 2021/219801 A1 is rather complex. Manufacture of the tip is also a bit time consuming in that the potting must be added in a controlled manner by trained operators to avoid bubble formation, spilling etc. Further curing may be time consuming, especially if the potting cannot be completely cured by UV curing, e.g. due to shadow effects. Heat curing at elevated temperatures, e.g. 110 degrees C for extended time, such as 60 minutes, could potentially have a negative impact on some electronic components.

Another document WO 2020/006158 A1 discloses a camera module in which optical and electrical components are fully encapsulated by an encapsulation material. In particular, the optical and electrical components are placed in a forming mold and the encapsulation material is injected into the forming mold. The camera module disclosed in WO 2020/006158 A1 has the disadvantages that the injection and/or curing of the encapsulation material may destroy or wear the sensitive electrical and optical components, e.g. due to heat generated during curing. Further, it is difficult to precisely control position of the components in the mold and on top there is the danger of undesired displacement of the electrical and optical components during injection and/or curing. Additionally, it is not possible to provide further functions inside the housing, such as light guides or light blocks. Molding is found to be difficult to control, so uniform production is difficult to achieve, e.g. because of bubble formation causing holes or other defects, necessitating careful inspection and manual remedy, which is time consuming. The mold (e.g. made of silicone) is worn quickly, such as after production of 4-20 parts.

### Brief description of the disclosure

The tasks and objectives of the present disclosure are to eliminate or at least to reduce the disadvantages of the related art. In particular, an endoscope shall be provided, which is designed for single-use and which provides improved quality control and assembly. In particular, it is desirable to avoid as far as possible that the entire endoscope needs to be scrapped in case of a specific defect in the distal tip unit, in particular in the imaging device and the light emitting device, relating to e.g. liquid-tightness, quality and functionality. Further, it shall be prevented that the sensitive electrical and optical components are displaced, worn or even destroyed during manufacturing and/or assembly.

The tasks and objectives of the present disclosure are solved by an endoscope in accordance with claim 1 and by a system in accordance with claim 15. Advantageous embodiments are claimed in the dependent claims and/or are explained below.

The present disclosure relates to an endoscope comprising: a proximal endoscope handle or interface; and an insertion cord configured to be inserted into a patient's body cavity and comprising a modular distal tip unit; the modular distal tip unit comprising: a main body connected to a remainder of the insertion cord; and a camera module configured to be accommodated and inserted in a receptacle formed in the main body, the camera module comprising a non-cylindrical housing defining an internal hollow space in which at least a light emitting device and an imaging device are accommodated.

Said differently, the present disclosure provides a modular design of the distal tip unit. The camera module according to the present disclosure is in particular a separate and independent module or unit, which comprises at least the light emitting device and the imaging device and which can be tested, e.g. for quality, functionality, liquid tightness, etc. alone, i.e. without being built into the main body of the distal tip unit and thus without being built into the endoscope. It is thus possible to test and approve the camera module in a state in which the camera module is separate from, i.e. not assembled in an endoscope. The camera module tested and approved can be kept on stock and mounted in an endoscope when needed. In case any camera module does not pass a certain test only said camera module needs to be scrapped and not the entire finished endoscope. Therefore, it is possible to lower costs and scrap. The present disclosure enables improved quality control and easier assembly due to the modular configuration of the distal tip unit. A further advantage of providing the modular distal tip unit is that different functions of the endoscope can be split up on different parts, so each module may be relatively simple to design, produce and assemble. The risk of problems with the quality is hence reduced compared to a more complex design.

It is to be understood that "proximal" means close to an operator/ user and away from a patient and "distal" means away from the operator/ user and close to the patient according to the present disclosure.

The endoscope according to the present disclosure is preferably a low-cost, lightweight, single-use endoscope, which is intended to be disposed after use. This means that the endoscope is preferably optimized for one single use. The endoscope preferably has a limited number of elements, which are preferably manufactured with a low-cost material (polymer/ plastic/ resin) in preferably a low-cost manufacturing process (plastic/ injection molding) and which can be easily assembled. Compared to traditional reusable endoscopes, the focus of the present disclosure is to provide an endoscope which is only used once and which thus does not have to withstand rather aggressive cleaning or sterilization processes and general harsh handling over the life cycle of the endoscope.

The endoscope according to the present disclosure is preferably a duodenoscope, i.e. is preferably optimized for endoscopic examination of the duodenum. Said differently, the modular distal tip unit according to the present disclosure is preferably applied in a duodenoscope. However, the present disclosure is not limited to the endoscope being a duodenoscope. In particular, the endoscope according to the present disclosure may advantageously also be another specialized medical instrument like a bronchoscope, arthroscope, colonoscope, laparoscope, gastroscope, ureteroscope, etc.

The insertion cord of the endoscope preferably comprises an insertion tube, a bending section as an actively bendable section and the modular distal tip unit extending in this order in the proximal-distal direction. The bending section may comprise a plurality of bending segments, in particular a proximal end segment, a plurality of intermediate segments and a distal end segment, e.g. connected to each other via flexible hinge members. In case the endoscope is set up like this the main body of the distal tip unit is preferably connected to the bending section, in particular to the distal end segment of the bending section. However, it is not necessary that the endoscope comprises an actively bendable bending section. Said differently, the insertion cord may be flexible without being actively bendable, or may alternatively be stiff according to the present disclosure.

The main body of the distal tip unit may be connected to the remainder of the insertion cord, in particular to the bending section by glue. A cover sleeve enclosing the bending section may also be connected to the main body, in particular by glue.

The main body of the modular distal tip unit preferably comprises a first main body housing part and a second main body housing part connected, in particular attached, to each other. Especially preferred, the first main body housing part is connected, in particular attached, to the remainder of the insertion cord, e.g. via an annular connection portion provided on a proximal end of the first main body housing part. The first main body housing part, in particular its annular connection portion may be connected to the remainder of the insertion cord, in particular to the bending section by glue.

Preferably, the receptacle in which the housing of the camera module is inserted and accommodated is formed in the first main body housing part. The second main body housing part may be formed as a cover or lid. The main body is not limited to having only or exactly the first main body housing part and the second main body housing part as housing parts. E.g., there may be provided further housing parts like side cover housing parts etc. Further, there may be provided only one single monolithically made housing part, i.e. not a multi-part housing.

The main body, in particular the first main body housing part, may comprise an opening, in which a protruding/ projecting portion of the camera module (housing) is arranged and accommodated. This makes it possible that a portion of the camera module, in particular the protruding/ projecting portion forms part/ is provided at the outer circumference of the distal tip unit. It is thus possible by suitable arrangement of the imaging device and the light emitting device inside the camera module that the patient's body cavity can be illuminated by the light emitting device and pictures/ videos can be taken by the imaging device. Moreover, the opening in the main body, in particular the first main body housing part, and the protruding/ projecting portion of the camera module (housing) adapted in size and shape to the opening make a defined arrangement of the camera module (housing) in the receptacle of the main body possible.

The defined arrangement is preferably further improved by providing at least one pinhole in the camera module (housing), into which a pin provided in the main body, in particular in the first main body housing part, is inserted, when the camera module (housing) is inserted into the receptacle of the main body.

Moreover, the defined arrangement of the camera module (housing) in the main body may be further improved by providing at least one rib, preferably several ribs or a rib structure, in the main body, in particular in the second main body housing part. The at least one rib may contact or push the camera module (housing) from a side opposite the protruding/ projecting portion.

Especially preferred, the defined arrangement of the camera module (housing) is reached by the combination of the above-described measures, i.e. by arranging the protruding/ projecting portion of the camera module (housing) in the opening of the first main body housing part and inserting the pin provided in the first main body housing part into the pinhole provided in the camera module (housing) and contacting or pushing the camera module (housing) by the at least one rib provided in the second main body housing part.

Glue may be applied to fix the camera module (housing) in the defined arrangement. E.g., glue may be applied to the first main body housing part, in particular to one or more contact areas of the first main body housing part with the camera module (housing). In addition or alternatively, glue may be applied in the pinhole of the camera module (housing) in which the pin of the first main body housing part is arranged. In addition or alternatively glue may be applied to the at least one rib of the main body, in particular of the second main body housing part, contacting the camera module (housing).

The endoscope is preferably a side-viewing endoscope. I.e. the camera module (housing) is preferably arranged in the main body such that the endoscope is a side-viewing endoscope, i.e. not a front-viewing endoscope. This may be e.g. reached by providing an essentially cylindrically-shaped main body of the modular distal tip unit having a cylindrical shell surface portion and a distal-most tip surface portion, wherein the opening in the main body, in particular in the first main body housing part, is provided in the cylindrical shell surface portion.

The main body, in particular the first main body housing part, of the modular distal tip unit may form or comprise a working channel for inserting a tool or instrument into the patient's body cavity. I.e. the working channel is preferably not formed or comprised in the camera module but in the main body.

An elevator or actuating lever (so-called Albarran lever) may be provided or arranged in the main body (and thus not in the camera module). The elevator or actuating lever may be actuatable and may be pivoted by the operator/ user in order to direct the tool or the instrument inserted into the patient's body cavity via the working channel into a defined direction. The elevator or actuating lever may be pivotable such that in a fully pivoted position of the elevator the tool or instrument inserted into the patient's body cavity is deflected by around 90° with respect to an extension direction of the working channel/ the insertion cord of the endoscope.

The side-viewing capability of the endoscope is preferably such that an optical axis of the imaging device is or extends substantially or approximately parallel and in essentially the same radial direction as the instrument or tool deflected by the elevator in the fully pivoted position of the elevator, or that the optical axis is arranged so that a distal tip of an instrument or tool deflected by the elevator in the fully pivoted position of the elevator and extending about 20 mm is inside the view of the imaging device.

The main body may further comprise a rinsing channel and/or an insufflation channel. The rinsing channel and/or the insufflation channel are thus preferably not provided in the camera module. The rinsing channel may be provided to provide water under pressure for rinsing a soiled window of the light emitting device and/or the imaging device. The insufflation channel may be provided to provide air under pressure for inflating an organ like the duodenum, intestine, stomach or the like.

The camera module (housing) and the working channel may have a main extension direction extending in the proximal-distal direction, i.e. along the longitudinal axis of the insertion cord. The camera module (housing) and the working channel are thus preferably arranged next to each other seen in a cross-section of the modular distal tip unit. Especially preferred, in case the rinsing channel and/or the insufflation channel are comprised in the main body of the distal tip unit, the rinsing channel and/or the insufflation channel may also have a main extension direction extending in the proximal-distal direction and may be arranged next to each other and next to the working channel and the camera module seen in the cross-section of the modular distal tip unit.

According to the present disclosure, the housing of the camera module is non-cylindrical. I.e. the housing of the camera module in its entirety preferably has a non-rotationally symmetric outer contour. There may however be provided cylindrical portions in the housing as long as the housing in its entirety is non-cylindrical. The housing being non-cylindrical in particular means that no part encompassing the housing and being adapted to the shape of the housing can be both rotated and axially displaced with respect to the housing. An advantage of providing a non-cylindrical housing is that this facilitates correct positioning and alignment of the camera module in the main body.

According to an embodiment, the housing of the camera module has essentially or approximately a box shape having a length, a width and a height, wherein the width and the height are small compared to the length. E.g. the length (extension) may be between 15 mm and 30 mm, the width (extension) may be between 4 mm and 7 mm, and the height (extension) may be between 4 mm and 7 mm. The camera module is thus preferably a quite small and compact module. The walls may be parallel in pairs but need not to be.

Preferably, the housing of the camera module has a width extension and/or a height extension smaller than a radius of the insertion cord/ of at least a portion like the annular connection portion of the main body of the distal tip unit. The diameter of the insertion cord/ of the annular connection portion may e.g. be between 12 mm and 15 mm.

The camera module has a size and/or shape, in particular a width and height, which makes it possible that there is space for components like the working channel, the elevator, the insufflation channel or the rinsing channel next to the camera module in the main body seen in the cross-section.

The housing defines an internal hollow space according to the present disclosure. I.e. there is a cavity/ hollow inside the housing. Therefore, electrical and optical components provided in the internal hollow space are not fully encapsulated by a potting/ encapsulation material. There is thus basically air in the internal hollow space. The provision of the housing having the internal hollow space has the advantages that the electrical and optical components arranged inside the housing may be suitably positioned inside the housing and are not worn/ destroyed/ displaced during assembly and/or manufacturing.

An entirety of, i.e. all electrical and optical elements or components, including the light emitting device and the imaging device, provided in the modular distal tip unit are preferably provided/ accommodated in the internal space of the housing of the camera module. I.e. preferably no electrical and optical elements or components are arranged (elsewhere) in the main body.

Preferably, the entirety of cables, which have to be guided out from the housing of the camera module, are guided into the (one single) cable sleeve. The cable sleeve is preferably a part of the camera module. The cable sleeve preferably extends through the entire insertion cord into the proximal endoscope handle or interface. The cables may be guided out of the cable sleeve in the proximal endoscope handle or interface and may be connected to a handle or interface printed circuit board, which is a printed circuit board arranged in the proximal endoscope handle or interface. Such a handle or interface printed circuit board may also be considered to be a part of the camera module.

According to a preferred embodiment, the camera module may comprise a cable sleeve liquid-tightly attached to the housing of the camera module. In particular, the housing of the camera module may comprise a connector part or portion to which the cable sleeve is liquid-tightly attached. The cable sleeve may e.g. be inserted into the connector part or may be mounted onto, e.g. pulled over, the connector part.

According to a preferred embodiment, the housing of the camera module comprises a cable lead-in bushing as connector part or portion, and the cable sleeve is liquid-tightly attached to the cable lead-in bushing.

The cable sleeve may be mounted over a reinforcing (metal) tube, and the cable sleeve and the reinforcing tube may be inserted into and accommodated in the cable lead-in bushing provided in the housing of the camera module. By providing the reinforcing tube, it is possible to expand the cable sleeve. The reinforcing tube is preferably adapted to the cable lead-in bushing such that a tight fit to an inner wall (surface) of the cable lead-in bushing is reached. In this case, the reinforcing tube preferably provides a reinforcement of the cable lead-in bushing.

The cable lead-in bushing is further preferred transparent, i.e. is a transparent portion of the housing of the camera module. By forming the cable lead-in bushing with a transparent material it is possible to use UV glue, which may be added to the cable-lead-in bushing, in order to reach the desired liquid tight connection between the cable lead-in bushing and the cable sleeve.

Alternatively, the connector part or portion may be a pipe connection and the cable sleeve may be mounted onto, i.e. pulled over on the outside over the pipe connection and preferably glued to the same.

The housing of the camera module may comprise a first (top) camera housing part and a second (bottom) camera housing part liquid-tightly connected/ attached to each other. The first camera housing part may be formed as a receptacle for the electrical and optical elements. The second camera housing part may be essentially formed as a lid or cover. The first camera housing part and the second camera housing part may be glued or welded to each other. I.e. there is preferably provided a bond between the first and second camera housing parts, via which the desired liquid-tightness is reached. E.g., the first and second camera housing parts may be connected to each other via ultrasonic welding. In this case, it may be preferable to provide pointed edges between the first and second camera housing parts in order to increase the available surface for the ultrasonic welding. Alternatively, the first and second camera housing parts may be UV glued to each other. In this case, it may be preferable to provide a gap between the first camera housing part and the second camera housing part so that glue can flow into said gap in order to provide the seal for liquid tightness.

The first camera housing part and the second camera housing part are preferably injection-molded parts. The first camera housing part is especially preferred a two-component (2K) injection-molded part. The first camera housing part is preferably opaque, e.g. black, for the most part and has at least one transparent area or portion, preferably at least two transparent areas or portions. When the first camera housing part is essentially opaque, it is possible to avoid that stray light reaches the camera/ image sensor, thereby disturbing the picture.

A first transparent area or portion of the first camera housing part preferably forms the cable lead-in bushing, so that it is possible to use UV glue to liquid-tightly attach the cable sleeve to the cable lead-in bushing. Using UV glue has the advantage that curing can take place relatively quickly.

A second transparent area or portion of the first camera housing part preferably forms a window for the light emitting device and/or for the imaging device. There may be provided one connected transparent area or portion for both the light emitting device and the imaging device, which is preferred according to the present disclosure. Alternatively, there may be provided two separate transparent areas or portions, i.e. second and third transparent areas or portions, one for the light emitting device and one for the imaging device.

In case the first camera housing part and the second camera housing part are connected via UV glue, the second camera housing part is preferably entirely made from a transparent material. In case ultrasonic welding is used for connecting the first camera housing part and the second camera housing part, the second camera housing part may be made from a transparent material or alternatively from an opaque, e.g. black, material. Using an opaque material would be advantageous since it would be avoided that any light reaches the camera/ image sensor through the second camera housing part. Using a transparent material would be advantageous since it would be possible to visually check the inside of the camera module housing.

According to the present disclosure it is especially preferred if the camera module has a liquid-tight housing. In particular, liquid penetrating into the housing would have a negative impact on the performance of the light emitting device and the imaging device. Further, the provision of a liquid-tight housing minimizes a potential risk of electrical disturbances and discharges.

A liquid-tight housing is especially reached according to the present disclosure by providing a liquid-tight connection between the first camera housing part and the second camera housing part and by providing a liquid-tight connection between the connector part or portion of the camera module housing, in particular of the first camera housing part, and the cable sleeve.

It is to be understood that the cable sleeve, in particular an inner lumen of the cable sleeve, is preferably open at its proximal end, which is in particular arranged in the proximal endoscope handle or interface in the assembled state of the endoscope, and is preferably open from its proximal end to its distal end attached to the housing of the camera module. Therefore, it is possible to test the camera module housing in a state disassembled or separate from the remainder of the endoscope for liquid tightness as follows: The camera module housing can be put or submersed into water and air can be blown into the proximal end of the cable sleeve. In case no bubbles are visible in the water, the camera module housing is liquid-tight. It thus applies that not the entire camera module is liquid-tight since liquid could theoretically penetrate into the camera module housing via the cable sleeve. However, when only the camera module housing is subjected to water and not the entire camera module, which is usually the case in practice since the proximal open end of the cable sleeve is preferably arranged in the proximal endoscope handle or interface, the camera module housing may be defined as liquid-tight according to the present disclosure. The camera module may in addition also be tested for electrical robustness in the state disassembled or separate from the remainder of the endoscope. E.g. the so-called HIPOT test, which is a dielectric strength test, can be performed. Therefore, it is possible according to the present disclosure to only install camera modules into endoscopes, which have already passed all necessary tests like the test for liquid-tightness, the HIPOT test, etc.

According to the present disclosure there are provided at least the light emitting device and the imaging device in the internal space defined by the housing of the camera module. The light emitting device is preferably a light emitting diode (LED). A light emitting diode is preferred as light emitting device, since it requires only small space, is robust, does not require large amounts of energy and provides light of high quality well suited for medical examination and surgery. The imaging device preferably comprises an image sensor/ camera sensor/ imaging chip and a focusing system/ lens system like a lens barrel.

Preferably, there is also provided a (camera module) printed circuit board in the internal hollow space of (inside) the housing of the camera module. The light emitting device and/or the imaging device are preferably arranged on and/or connected, in particular electrically connected, to the (camera module) printed circuit board inside the housing of the camera module.

The printed circuit board, the light emitting device and the imaging device are preferably fitted or inserted into the first camera housing part.

The (camera module) printed circuit board may be a folded printed circuit board so that the available internal hollow space defined by the housing of the camera module can be suitably and optimally used. The printed circuit board being a folded printed circuit board preferably means that the printed circuit board comprises at least two parallel printed circuit board planes/ portions. Therefore, it is possible to suitably arrange the printed circuit board in the internal space of the housing of the camera module and to suitably use the available internal space, which is in particular advantageous in the case of a compact, e.g. essentially box shaped, camera module housing, which has a rather small width and/or height. Moreover, the provision of a folded printed circuit board may have the advantage that the printed circuit board has a certain flexibility, which may be used for positioning the imaging device and/or the light emitting device correctly with respect to each other and/or with respect to a window provided in the housing of the camera module.

Preferably, the (all) cables attached to the (camera module) printed circuit board, which need to be guided to the endoscope handle or interface or potentially further to a display unit/ monitor connected to the endoscope handle or interface, are drawn through the one single cable sleeve. The cables thus preferably extend inside the cable sleeve to the proximal endoscope handle or interface. Further preferred, the (all) cables are attached to a proximal portion of the (folded) printed circuit board.

A shielding element like a copper foil may be wrapped around the (camera module) printed circuit board. The shielding element may contribute to electromagnetic compatibility. The shielding element may also help to avoid that high frequency tools or instruments inserted into the working channel of the endoscope affect the electrical or optical elements provided in the camera module, e.g. the camera/ image sensor.

The present disclosure further relates to a system comprising an endoscope as described above and a monitor connectable to the endoscope.

According to a further aspect, which may be independently claimed, potentially in combination with any of the above described aspects and/or the dependent claims, the present disclosure relates to an endoscope comprising: a proximal endoscope handle or interface; and an insertion cord configured to be inserted into a patient's body cavity and comprising a modular distal tip unit; the modular distal tip unit comprising: a main body connected to a remainder of the insertion cord; and a camera module configured to be accommodated and inserted in a receptacle formed in the main body, the camera module comprising a housing defining an internal hollow space in which at least a light emitting device and an imaging device are accommodated.

### Brief description of figures

The disclosure is explained in more detail below using preferred embodiments and referring to the accompanying figures.
- Fig. 1: shows a side view of an endoscope according to the present disclosure;
- Fig. 2: shows a perspective view of a distal tip unit according to the present disclosure;
- Fig. 3: shows a perspective view of the distal tip unit illustrating an assembly of a camera module housing;
- Fig. 4: shows another perspective view of the distal tip unit illustrating an assembly of the camera module housing;
- Fig. 5: shows a perspective longitudinal sectional view of the distal tip unit and its connection to a bending section;
- Fig. 6: shows a camera module according to the present disclosure in a disassembled state;
- Fig. 7: shows a perspective detailed view of the camera module housing illustrated in Fig. 6 from the top;
- Fig. 8: shows a perspective detailed view of the camera module housing illustrated in Fig. 6 from the bottom;
- Fig. 9: shows a perspective view of the camera module housing in a state disassembled from a cable sleeve;
- Fig.10: shows a perspective longitudinal sectional view of the camera module housing illustrating the components provided inside the same.
- Fig. 11: shows a first perspective view of the components provided inside the camera module housing; and
- Fig. 12: shows a second perspective view of the components provided inside the camera module housing.

The figures are schematic in nature and serve only to understand the disclosure. Identical elements are marked with the same reference signs.

### Detailed description of preferred embodiments

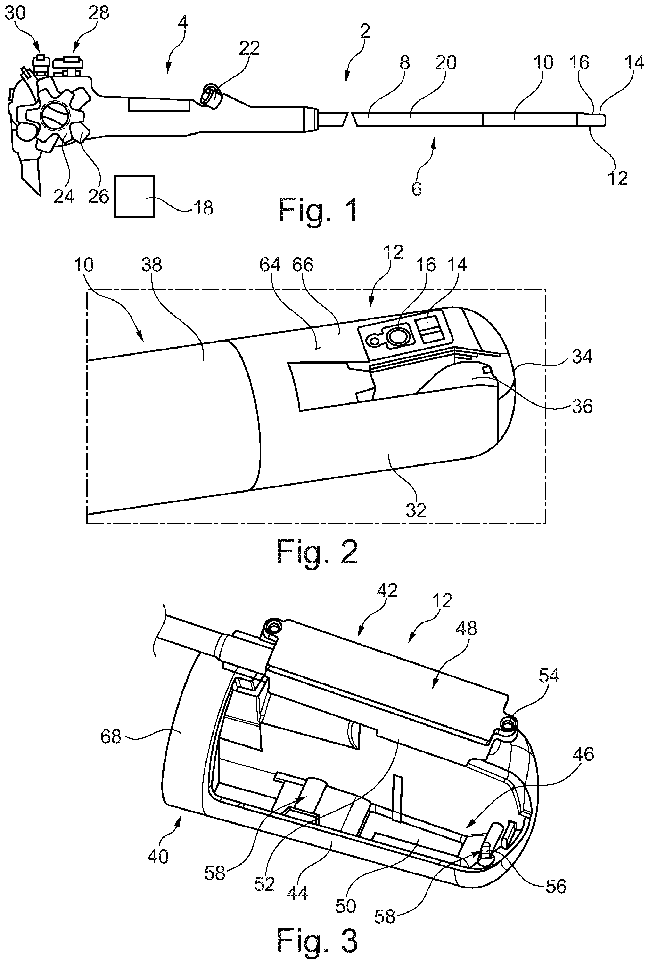

In Fig. 1, an endoscope 2 is shown. The endoscope 2 is preferably a single-use endoscope. The endoscope 2 comprises a proximal endoscope handle 4 designed to be held by a user and being configured to accommodate operating parts of the endoscope 2. The endoscope 2 further comprises an insertion cord 6 configured to be inserted into a patient's body cavity. The insertion cord 6 comprises an insertion tube 8, a bending section 10 and a modular distal tip unit 12, which extend in this order from the endoscope handle 4. In the modular distal tip unit 12 a light emitting device 14 and an imaging device 16 are accommodated, so that the patient's body cavity can be illuminated and inspected. Pictures/ videos captured by the imaging device 16 can be shown on a monitor 18 provided separately from and connectable with the endoscope 2.

The endoscope 2 has an internal working channel 20, which is accessible via an access port 22 and via which a surgical tool or instrument can be guided into the patient's body cavity.

The endoscope handle 4 comprises two handle wheels, namely a first handle wheel 24 and a second handle wheel 26, which are arranged coaxially and which can be rotated by the user in order to bend the bending section 10 for steering the modular distal tip unit 12. In particular, one of the handle wheels 24, 26, e.g. the first handle wheel 24, can be operated by the user to bend the bending section 10 in a first bending plane (e.g. in an up-and-down direction) and the other one of the handle wheels 24, 26, e.g. the second handle wheel 26, can be operated by the user to bend the bending section 10 in a second bending plane (e.g. in a right-and-left direction). The first bending plane is preferably perpendicular to the second bending plane. The endoscope 2 shown in Fig. 1 is thus a two-plane bending endoscope.

The endoscope handle 4 further comprises two valves, namely a gas/water injection valve 28 and a suction valve 30. The gas/water injection valve 28 and the suction valve 30 are arranged side by side on a surface of a housing of the endoscope handle 4. The gas/water injection valve 28 is preferably connected to both a gas/air source, in particular a gas cylinder/ bottle (preferably filled with carbon dioxide), and a water source, in particular a water tank/ bottle, via a connector unit. An insufflator may be provided to pump both gas and water into the endoscope 2. The gas/ water injection valve 28 can be brought into different positions/ states, in order to reach different purposes. In particular, the gas/water injection valve 28 can be controlled such that gas, in particular CO2, is provided to an insufflation channel provided in the insertion cord 6, or controlled such that water is provided to a rinsing channel provided in the insertion cord 6. The suction valve 30 is preferably connected to the working channel 20 and - via the connector unit - to a suction device like a vacuum pump. The suction valve 30 can be brought into a valve closed state, in which no suction is applied to the working channel 20 and to a valve open state, in which suction is applied to the working channel 20.

Fig. 2 shows a perspective view of the modular distal tip unit 12 according to the present disclosure. The modular distal tip unit 12 is essentially cylindrically-shaped and has a cylindrical shell surface portion 32 and a distal-most tip surface portion 34. The light-emitting device 14 comprising two light-emitting diodes and the imaging device 16 are provided in the cylindrical shell surface portion 32. The endoscope 2 according to the present disclosure is thus preferably a side-viewing endoscope 2. In the modular distal tip unit 12, an elevator 36 (so called Albarran lever) is provided, which can be actuated and pivoted by the user via a lever or wheel provided at the endoscope handle 4, in order to direct a tool or instrument inserted into the patient's body cavity via the working channel 20 into a defined direction. In a fully pivoted position of the elevator 36 the tool or instrument is deflected by around 90° with respect to an extension direction of the working channel 20. An optical axis of the imaging device 16 extends substantially parallel with and in the same radial direction as the instrument or tool deflected by the elevator 36 in its fully pivoted position. Fig. 2 further shows an end portion of the bending section 10, which comprises a flexible cover sleeve 38 for preventing contamination.

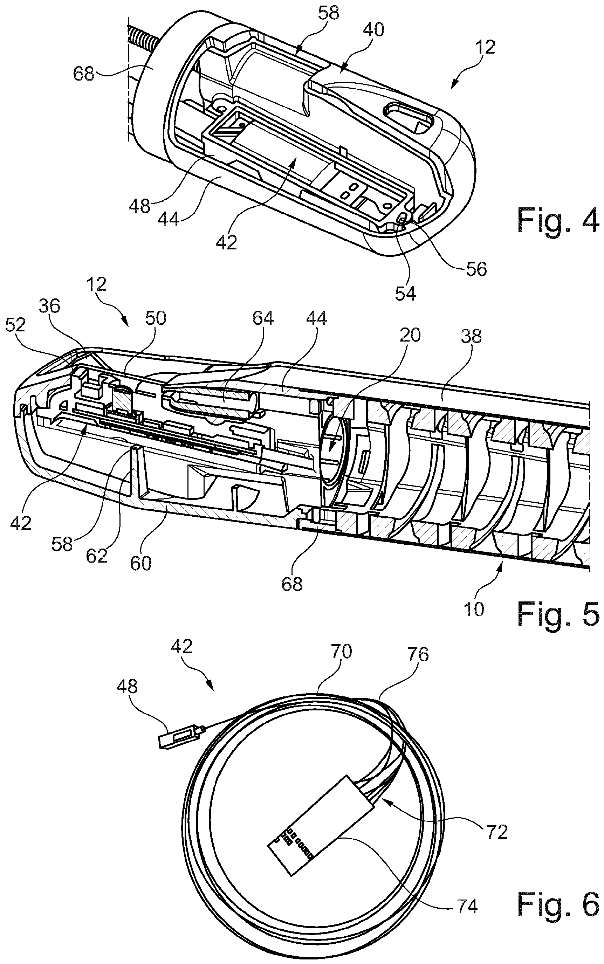

The modular construction of the modular distal tip unit 12 is illustrated in Fig. 3, Fig. 4 and Fig. 5. In Fig. 3 and Fig. 4 assembly steps of the modular distal tip unit 12 are shown. Fig. 5 shows a longitudinal sectional view of the modular distal tip unit 12 and its connection to the bending section 10.

The modular distal tip unit 12 according to the present disclosure basically comprises a main body 40 connected to a remainder of the insertion cord 6, in particular to the bending section 10, and a (portion of a) camera module 42. The main body 40 comprises a first main body housing part 44, which forms a receptacle 46 for a housing of the camera module 42, which is designated as camera module housing 48 in the following.

Fig. 3 shows the first main body housing part 44 and the camera module 42 in a disassembled state. The first main body housing part 44 comprises an opening 50 which is adapted in size and shape to a projecting portion 52 of the camera module housing 48. In order to arrange the camera module housing 48 in a defined way in the first main body housing part 44, the projecting portion 52 of the camera module housing 48 is inserted into the opening 50. Further, a pinhole 54 is provided in the camera module housing 48 into which a pin 56 provided in the first main body housing part 44 is inserted for position of the camera module housing 48. Fig. 3 further shows glue application areas 58 on a bottom surface of the receptacle 46 and in an area around/ at the pin 56, where glue is applied in order to fix the camera module housing 48 to the first main body housing part 44.

Fig. 4 shows the modular distal tip unit 12 in a state in which the camera module housing 48 is already arranged and accommodated in the first main body housing part 44. In this state, glue may be applied at the glue application area 58 shown in Fig. 4 and the second main body housing part 60 (not shown in Fig. 4) of the main body 40 may be fixed to the first main body housing part 44.

Fig. 5 shows the modular distal tip unit 12 in a fully assembled state, i.e. with the second main body housing part 60 being attached to the first main body housing part 44 by glue applied at the glue application area 58 along the rim of the opening as seen in Fig. 4. Glue may be applied along the whole rim, but this is not necessarily the case, as the main focus is that the second main body housing part 60 should not disentegrate from the first main body housing part 44, whereas fluid tightness is not mandatory, especially for single-use endoscopes, where reprocessing is not an issue. With reference to Fig. 4 and Fig. 5 it can be seen that the second main body housing part 60 essentially forms a cover or lid. The second main body housing part 60 comprises a rib 62, which may be glued to the camera module housing 48 at another glue application area 58.

As can be further seen in Fig. 5, the first main body housing part 44 forms (a portion of) the working channel 20. The working channel 20 comprises in addition to the portion provided in the first main body housing part 44 a (not shown) working channel tube, which is connected to said portion and extends through the bending section 10 and the insertion tube 8, and a (not shown) biopsy connector/ Y-connector provided in the endoscope handle 4. The elevator 36 is provided at a distal end of the working channel 20 for deflecting the tool or instrument inserted into the patient's body cavity. A rinsing channel 64 is further shown in Fig. 5. Next to the rinsing channel 64, there is provided an insufflation channel 66, which is schematically indicated in Fig. 2.

Fig. 5 further shows the connection of the modular distal tip unit 12 to the bending section 10. In particular, the first main body housing part 44 comprises an annular connection portion 68, which is glued to a distal end segment of the bending section 10. The bending section 10 basically comprises a plurality of bending segments connected to each other via flexible hinge members. The cover sleeve 38 is also glued to the first main body housing part 44.

Fig. 6 shows the camera module 42 in a state not assembled into an endoscope 2, i.e. as a separate module. The camera module 42 basically comprises the camera module housing 48, a cable sleeve 70, into which an entirety of cables 72, which have to be guided out from the camera module housing 48, are guided into, and a handle printed circuit board 74. The cable sleeve 70 is a long, sleeve-like, hollow cylindrical component. In a state in which the camera module 42 is assembled into the endoscope 2, the cable sleeve 70 extends through the entire insertion cord 6 into the proximal endoscope handle 4, in which the handle printed circuit board 74 is arranged. Fig. 6 further shows an end of the cable sleeve 70, which is designated as cable sleeve end 76 in the following. The entirety of cables 72 leave the cable sleeve 70 at the cable sleeve end 76 inside the endoscope handle 4.

The configuration of the camera module 42 as shown in Fig. 6 makes it possible to test the camera module housing 48 for liquid tightness as follows: The camera module housing 48 and a distal end portion of the cable sleeve 70 can be submersed into water and air can be blown into the cable sleeve end 76. In case no bubbles are visible in the water, the camera module housing 48 is liquid-tight. Further necessary tests like the HIPOT test can be performed in the disassembled state of the camera module 42 shown in Fig. 6.

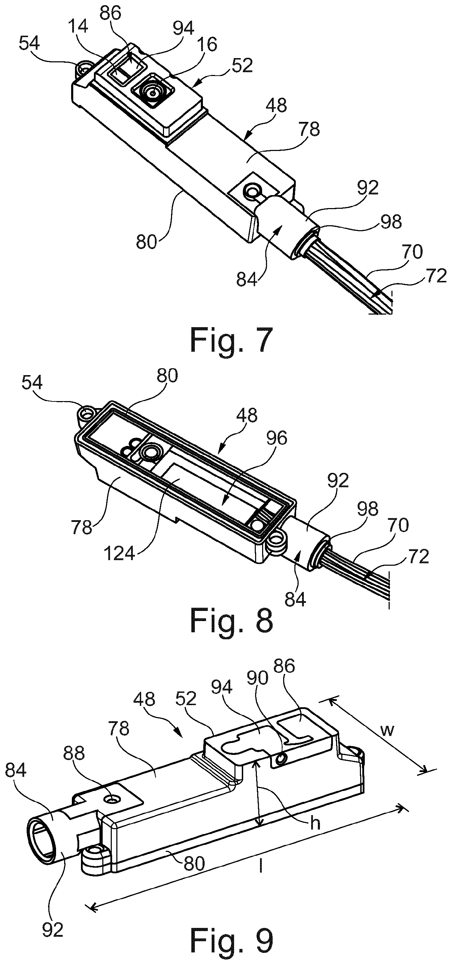

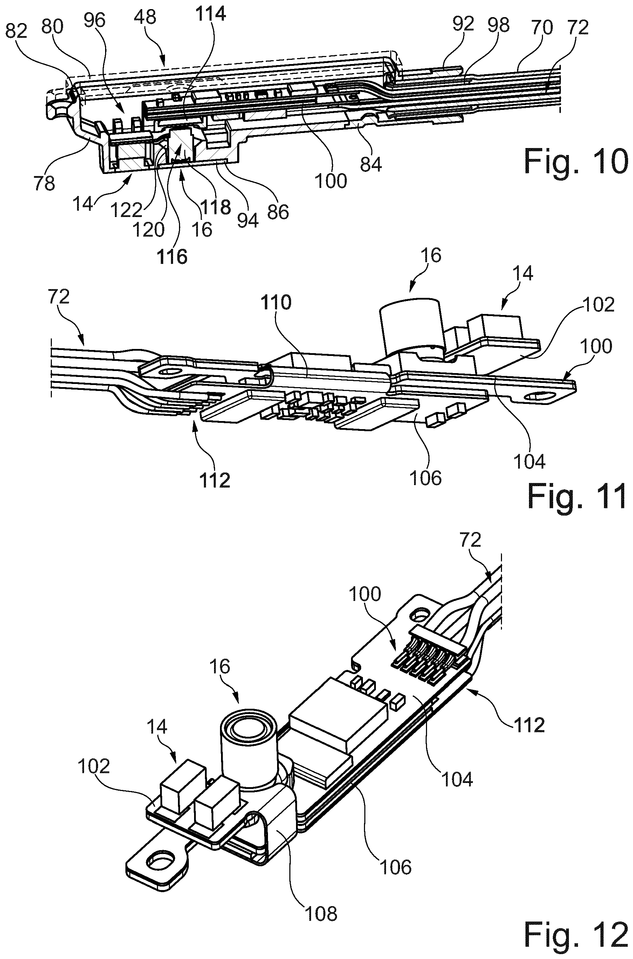

The configuration of the camera module housing 48, its connection to the cable sleeve 70 and the components provided inside the camera module housing 48 are illustrated in Fig. 7, Fig. 8, Fig. 9 and Fig. 10. Fig. 7 is a perspective detailed view of the camera module housing 48 illustrated in Fig. 6 from the top. Fig. 8 is a perspective detailed view of the camera module housing 48 illustrated in Fig. 6 from the bottom. Fig. 9 is a perspective view of the camera module housing 48 in a state disassembled from the cable sleeve 70. Fig.10 is a perspective longitudinal sectional view of the camera module housing 48 illustrating the components provided inside the same.

As can be seen in particular in Fig. 7, Fig. 8 and Fig. 9, the camera module housing 48 is non-cylindrical and has an approximately box shape. In Fig. 9 maximum extensions in length I, height h and width w of the camera module housing 48 are shown. The width w and the height h are small compared to the length I. E.g. the maximum length extension I is between 15 mm and 30 mm, the maximum width extension w is between 4 mm and 7 mm, and the maximum height extension h is between 4 mm and 7 mm. The width w and the height h are preferably smaller than a radius of the insertion cord 6.

The camera module housing 48 comprises a first top camera housing part 78 and a second bottom camera housing part 80. The first top camera housing part 78 is formed as a receptacle for the components to be inserted into the camera module housing 48. The second bottom camera housing part 80 is formed as a lid or cover. The first top camera housing part 78 and the second bottom camera housing part 80 are liquid-tightly connected to each other via a bond, i.e. e.g. via gluing or welding. In the longitudinal sectional view of Fig. 10 it can be seen that the first top camera housing part 78 and the second bottom camera housing part 80 preferably comprise pointed edges 82 for facilitating ultrasonic welding. Alternatively, e.g. UV glue may be used for providing the connection between the first and second camera housing parts 78, 80.

The first top camera housing part 78 is a two-component injection-molded part. The first top camera housing part 78 is opaque, e.g. black, for the most part and has two transparent areas, namely a first transparent area 84 and a second transparent area 86. Fig. 9 shows injection-molding inlet points 88, 90 of the first and second transparent areas 84, 86, which are injection points of the transparent material used for the first and second transparent areas 84, 86. The first transparent area 84 comprises a cable lead-in bushing 92, which is to be described below, in particular its connection to the cable sleeve 70 and is provided at a proximal end of the camera module housing 48. The second transparent area 86 comprises or forms a window 94 for both the light emitting device 14 formed as two light-emitting diodes and the imaging device 16. The second transparent area 86 is provided at the projecting portion 52 of the camera module housing 48, which is rather at the distal end of the camera module housing 48. The second bottom camera housing part 80 is an injection-molded part formed from a transparent material according to a preferred embodiment, so that it is possible to visually check an internal hollow space 96 of the camera module housing 48.

As can be seen in Fig. 7, Fig. 8 and in particular Fig. 10, the cable sleeve 70 is mounted over a reinforcing metal tube 98, thereby expanding the cable sleeve 70, and the combination of cable sleeve 70 and reinforcing metal tube 98 is inserted into the cable lead-in bushing 92. The reinforcing metal tube 98, the cable sleeve 70 and the cable lead-in bushing 92 are adapted to each other such that a tight fit connection is provided when the combination of cable sleeve 70 and reinforcing metal tube 98 is inserted into the cable lead-in bushing 92. Since the cable lead-in bushing 92 is made from a transparent material, it is possible to add UV glue to the cable-lead-in bushing 92 or to the cable sleeve 70 for providing a liquid tight connection between the cable lead-in bushing 92 and the cable sleeve 70. The camera module housing 48 is liquid tight according to the present disclosure by providing a liquid-tight connection between the first top camera housing part 78 and the second bottom camera housing part 80 and by providing a liquid-tight connection between the cable lead-in bushing 92 and the cable sleeve 70.

All electrical and optical elements or components provided in the modular distal tip unit 12 are accommodated in the internal space 96 of the camera module housing 48. Fig. 10 shows the arrangement of the electrical and optical elements in the internal space 96 of the camera module housing 48. Fig. 11 and Fig. 12 show perspective views of the electrical and optical elements without the camera module housing 48. In the internal space 96 of the camera module housing 48 a camera module printed circuit board 100 is arranged. The light emitting device 14 formed as two light emitting diodes and the imaging device 16 are electrically connected to the camera module printed circuit board 100. A plurality of further electrical elements like a microcontroller, transistors, resistors, capacitors, etc. are provided on, respectively connected to the camera module printed circuit board 100, as known in the art.

The camera module printed circuit board 100 is a folded printed circuit board and comprises three parallel planes, namely a first plane 102, a second plane 104 and a third plane 106. As can be seen in Fig. 12, the first plane 102 and the third plane 106 are connected via a first folded portion 108, which extends perpendicular to the first plane 102 and the third plane 106. The folded portion 108 may have a certain extension giving flexibility of the position of the camera 16 and the light emitting device 14 relative to the rest of the camera module printed circuit board 100. Hereby it is possible to more accurately position the camera 16 and light emitting device 14 correct relatively to the window 94 as seen in Fig. 10. As can be seen in Fig. 11, the second plane 104 and the third plane 106 are connected via a second folded portion 110, which extends perpendicular to the second plane 104 and the third plane 106, parallel to the first folded portion 108, and is provided on a side opposite with respect to the first folded portion 108. In a height direction h of the camera module housing 48, the first plane 102 is arranged on top, i.e. closest to the window 94 provided by the second transparent area 86, the second plane 104 is arranged in the middle, and the third plane 106 is provided on the bottom. The first plane 102 is essentially only provided at a small distal portion (i.e. does not extend over a majority length portion) of the camera module housing 48 and only carries the light emitting device 14/ the two light emitting diodes. The second plane 104 and the third plane 106 extend over a majority length portion of the camera module housing 48. The imaging device 16 is arranged on the second plane 104 and in a proximal-distal direction, i.e. in a length direction of the camera module housing 48 close to/ directly proximally with respect to the light emitting device 14. The second plane 104 and the third plane 106 comprise the mentioned further electrical components, as can be seen in Fig. 10, Fig. 11 and Fig. 12. All cables 72, which are attached to the camera module printed circuit board 100, are attached to the same at a proximal end portion 112 of the camera module printed circuit board 100 and in particular both to the second plane 104 and the third plane 106. The cables 72 are all guided into the one single cable sleeve 70 as shown in Fig. 10, in which they are guided towards the proximal endoscope handle 4.

As can be in particular seen in Fig. 10, the imaging device 16 comprises an image/ camera sensor 114, which is located on and electrically connected to the camera module printed circuit board 100, and a lens system 116 for providing an image on the image/ camera sensor 114. An optical axis of the lens system 116 is, substantially, parallel to an optical axis of light emitted by the light emitting device 14. Therefore, both the optical axis of the imaging device 16/ the lens system 116 and the optical axes of the light emitting diodes of the light emitting device 14 are arranged orthogonal to the first plane 102 and the second plane 104 of the camera module printed circuit board 100. The lens system 116 comprises a stack of lenses 118 stacked in the direction of the optical axis thereby forming a so-called lens barrel, which is carried by the camera/ image sensor 114. Such a lens barrel - camera/ image sensor - unit is well known in the art so that a detailed description is omitted. The lens system 116 is formed cylindrically and is accommodated in a cylindrical accommodation portion 120 provided in the camera module housing 48, in particular in the first top camera housing part 78. The accommodating portion 120 is provided in close proximity around the lens system 116 so that there is hardly/ small space between the lens system 116 and the accommodation portion 120. The lens system 116 is glued to the cylindrical accommodation portion 120 via a glue portion 122, in order to isolate the small remaining air volume around the lens system 116. Therefore, a danger is reduced that condensed water gathers around the lens system 116, which might impair the images/ pictures taken by the imaging device 16, in particular when the light emitting device 14, which is close to the lens system 116, emits heat leading to condensation of the humidity provided inside the camera module housing, e.g. humidity stored in glues.

With reference again to Fig. 8, it can be seen that a copper foil 124 is wrapped around the camera module printed circuit board 100 in order to further improve electromagnetic compatibility.

List of reference signs
- 2: endoscope
- 4: endoscope handle
- 6: insertion cord
- 8: insertion tube
- 10: bending section
- 12: modular distal tip unit
- 14: light emitting device
- 16: imaging device
- 18: monitor
- 20: working channel
- 22: access port
- 24: first handle wheel
- 26: second handle wheel
- 28: gas/water injection valve
- 30: suction valve
- 32: cylindrical shell surface portion
- 34: distal-most tip surface portion
- 36: elevator
- 38: cover sleeve
- 40: main body
- 42: camera module
- 44: first main body housing part
- 46: receptacle
- 48: camera module housing
- 50: opening
- 52: projecting portion
- 54: pinhole
- 56: pin
- 58: glue application area
- 60: second main body housing part
- 62: rib
- 64: rinsing channel
- 66: insufflation channel
- 68: annular connection portion
- 70: cable sleeve
- 72: cables
- 74: handle printed circuit board
- 76: cable sleeve end
- 78: first top camera housing part
- 80: second bottom camera housing part
- 82: pointed edges
- 84: first transparent area
- 86: second transparent area
- 88: injection-molding inlet point
- 90: injection-molding inlet point
- 92: cable lead-in bushing
- 94: window
- 96: internal space
- 98: reinforcing metal tube
- 100: camera module printed circuit board
- 102: first plane
- 104: second plane
- 106: third plane
- 108: first folded portion
- 110: second folded portion
- 112: proximal end portion
- 114: image/ camera sensor
- 116: lens system
- 118: lens
- 120: accommodation portion
- 122: glue portion
- 124: copper foil

## Claims

1. Endoscope (2) comprising:
a proximal endoscope handle (4) or interface; and
an insertion cord (6) configured to be inserted into a patient's body cavity and comprising a modular distal tip unit (12);
the modular distal tip unit (12) comprising:
a main body (40) connected to a remainder of the insertion cord (6); and
a camera module (42) configured to be accommodated and inserted in a receptacle (46) formed in the main body (40), the camera module (42) comprising a non-cylindrical housing (48) defining an internal hollow space (96) in which at least a light emitting device (14) and an imaging device (16) are accommodated.

2. Endoscope (2) according to claim 1, wherein an entirety of electrical and optical components, including the light emitting device (14) and the imaging device (16), provided in the modular distal tip unit (12) are provided in the internal space (96) of the housing (48) of the camera module (42).

3. Endoscope (2) according to claim 1 or 2, wherein the housing (48) of the camera module (42) comprises a first camera housing part (78) and a second camera housing part (80) liquid-tightly attached to each other.

4. Endoscope (2) according to claim 3, wherein the first camera housing part (78) is a two-component injection-molded part being opaque for the most part and having at least one transparent area or portion (84, 86).

5. Endoscope (2) according to any one of the preceding claims 1 to 4, wherein the camera module (42) comprises a cable sleeve (70) liquid-tightly attached to the housing (48) of the camera module (42).

6. Endoscope (2) according to claim 5, wherein the cable sleeve (70) is mounted over a reinforcing tube (98), and the cable sleeve (70) and the reinforcing tube (98) are inserted into and accommodated in a cable lead-in bushing (92) provided in the housing (48) of the camera module (42).

7. Endoscope (2) according to claim 5 or 6, wherein an entirety of cables (72) guided out from the housing (48) of the camera module (42) are guided into the cable sleeve (70).

8. Endoscope (2) according to any one of the preceding claims 1 to 7, wherein a camera module printed circuit board (100) is provided in the internal space (96) of the housing (48) of the camera module (42), and the light emitting device (14) and the imaging device (16) are arranged on and connected to the camera module printed circuit board (100).

9. Endoscope (2) according to claim 8, wherein the camera module printed circuit board (100) is a folded printed circuit board.

10. Endoscope (2) according to any one of the preceding claims 1 to 9, wherein the housing (48) of the camera module (42) has a width extension (w) and/or a height extension (h) smaller than a radius of the insertion cord (6).

11. Endoscope (2) according to any one of the preceding claims 1 to 10, wherein the main body (40) of the modular distal tip unit (12) comprises a first main body housing part (44) and a second main body housing part (60) attached to each other, the first main body housing part (44) is attached to the remainder of the insertion cord (6), and the receptacle (46), in which the housing (48) of the camera module (42) is inserted and accommodated, is formed in the first main body housing part (44).

12. Endoscope (2) according to any one of the preceding claims 1 to 11, wherein the main body (40) of the modular distal tip unit (12) comprises an opening (50), in which a projecting portion (52) of the housing (48) of the camera module (42) is arranged and accommodated.

13. Endoscope (2) according to any one of the preceding claims 1 to 12, wherein the main body (40) of the modular distal tip unit (12) forms or comprises a working channel (20) for inserting a tool or instrument into the patient's body cavity.

14. Endoscope (2) according to any one of the preceding claims 1 to 13, wherein the endoscope (2) is a side-viewing endoscope.

15. System comprising: an endoscope (2) according to any one of claims 1 to 14; and a monitor (18) connectable to the endoscope (2).
